# EUROPEAN PATENT SPECIFICATION

(11) **EP 4 168 096 B1**
(45) Date of publication and mention of the grant of the patent: **06.05.2026**
(21) Application number: 21826948.8
(22) Date of filing: 17.06.2021
(51) Int. Cl.: A61M 60/17, A61M 60/274, A61M 60/438, A61M 60/451, A61M 60/454, A61M 60/515, A61M 60/837, A61M 60/861, A61M 60/863, A61M 60/865, A61F 2/00, A61F 2/24

(54) **RECONFIGURABLE FLUID DISPLACEMENT APPARATUSES AND METHODS OF USE**
REKONFIGURIERBARE FLÜSSIGKEITSVERDRÄNGUNGSVORRICHTUNGEN UND VERFAHREN ZUR VERWENDUNG
APPAREILS DE DÉPLACEMENT DE FLUIDE RECONFIGURABLES ET PROCÉDÉS D'UTILISATION

(30) Priority: 17.06.2020 US 202063040430 P
(43) Date of publication of application: 26.04.2023
(73) Proprietor: Najmabadi, Kamran, Palo Alto, California 94303 (US); Beygui, Ramin, Hillsborough, CA 94010 (US); Shamimi, Ali, Berkeley, CA 94705 (US)
(72) Inventor: Najmabadi, Kamran, Palo Alto, California 94303 (US); Beygui, Ramin, Hillsborough, CA 94010 (US); Shamimi, Ali, Berkeley, CA 94705 (US)
(74) Representative: IK-IP LTD
(86) International application number: PCT/US2021/037933
(87) International publication number: WO 2021/257904

(56) References cited:
- WO-A1-2018/068341
- WO-A1-2020/022905
- WO-A1-98/18508
- WO-A2-2011/056578
- US-A1- 2014 179 993
- US-B1- 9 623 163
- US-B2- 10 492 911
- US-B2- 10 639 145
- US-B2- 7 172 551
- US-B2- 7 758 491

## Description

### CROSS-REFERENCE TO RELATED APPLICATION

This application claims the benefit of U.S. provisional patent application serial no. 63/040,430, filed June 17, 2020.

### BACKGROUND

Heart failure occurs when the heart cannot pump enough blood for the needs of the body. Heart failure may be caused by a variety of conditions, including coronary artery disease, previous heart attacks, high blood pressure, damaged heart valves, and damaged heart muscle.

Ventricular assist devices may be used to supplement heart function. Some ventricular assist devices deliver continuous flow, which may introduce undesirable effects as the blood flow deviates from a normal, pulsatile flow. Other ventricular assist devices use pumps with high-speed rotating elements, which may damage red blood cells.

What is needed is a ventricular assist device that provides pulsatile flow. What is needed is a ventricular assist device that reduces damage to red blood cells.

US 2014/179993 discloses a foldable and expandable frame having at least one anchoring formation attached to an elongate manipulator and placed in a catheter tube while folded. The tube is inserted into a left ventricle of a heart where the frame is ejected from the tube and expands in the left ventricle. Movements of the elongate manipulator cause the anchor to penetrate the heart muscle and the elongate manipulator to release the frame. The installed frame minimizes the effects of an akinetic portion of the heart forming an aneurysmic bulge.

WO 2108/068341 discloses an intraventricular pulsatile blood pump that can be fixedly arranged at the cardiac apex inside a ventricle so as to generate an open-type pulsatile effect. The pulsatile blood pump is generally of a jellyfish shape, comprising a bell-shaped body and a driving source, with an opening of the bell-shaped body being opposite to an outlet of the ventricle, the driving source driving the bell-shaped body to perform systolic or diastolic movements. The systolic or diastolic movements of the bell-shaped body drive the blood inside the ventricle to be ejected directionally to an artery and to form a swirl type blood flow field between an inner wall of the bell-shaped body and an inner wall of the ventricle.

### SUMMARY

The invention is defined in the independent claim, with optional features being defined in the dependent claims. Ventricular assist devices configured to be placed in a ventricle of a heart are described. In one embodiment, a ventricular assist device comprises a pumping pouch having an opening and defining an internal volume configured to fill with blood in through the opening. The ventricular assist device further comprises a contraction element coupled to the contraction pouch and actuatable upon receipt of an actuation signal between first and second states in which force is exerted or not exerted inward on the pumping pouch such that when force is exerted inward on the pumping pouch, at least a portion of the pumping pouch is squeezed to force at least a portion of the blood out through the opening. The ventricular assist device further also comprises a frame including an upper ring coupled to the pumping pouch along an edge of the opening, the frame being configured to be coupled to a wall of the heart. The ventricular assist device further also comprises a controller electrically coupled to the contraction element, the controller comprising a processor configured to receive, in use, a signal from a sensor configured to sense beating of the heart and to synchronize actuation of the contraction element between the first and second states with the beating of the heart.

### BRIEF DESCRIPTION OF THE DRAWINGS

FIGURE 1 shows a ventricular assist device 1000 in place in a left ventricle of the heart.
FIGURE 2A shows one embodiment of ventricular assist device 1000 including a band 1210.
FIGURE 2B shows one embodiment of ventricular assist device 1000 including a helix 1220.
FIGURES 2C-2D show top and side views of one embodiment of ventricular assist device 1000 including a partial band 1230. FIGURE 2E shows partial band 1230 squeezing pumping pouch 1100.
FIGURES 2F-2G show top view and side views of one embodiment of ventricular assist device 1000 including a flap 1240. FIGURE 2G shows flap 1240 squeezing pumping pouch 1100.
FIGURE 3 shows one embodiment of ventricular assist device 1000 including a side strip 1250.
FIGURE 4 shows one embodiment of ventricular assist device 1000 including a bottom strip 1260.
FIGURE 5 shows one embodiment of ventricular assist device 1000 including a spring 1270.
FIGURE 6 shows a vertical cross-sectional view of ventricular assist device 1000.
FIGURES 7-8 show ventricular assist device 1000 in place in a left ventricle of a heart, with the heart in diastole and systole, respectively.
FIGURE 9 shows one embodiment of a delivery device 1600. FIGURE 10 shows a cross-sectional view of delivery device 1600, with ventricular assist device 1000 collapsed into a delivery configuration.
FIGURES 11-14 show one embodiment of a method for delivering ventricular assist device 1000 using delivery device 1600.
FIGURE 15 shows a vertical cross-sectional view of ventricular assist device 1000 in place in a left ventricle of the heart.

### DESCRIPTION

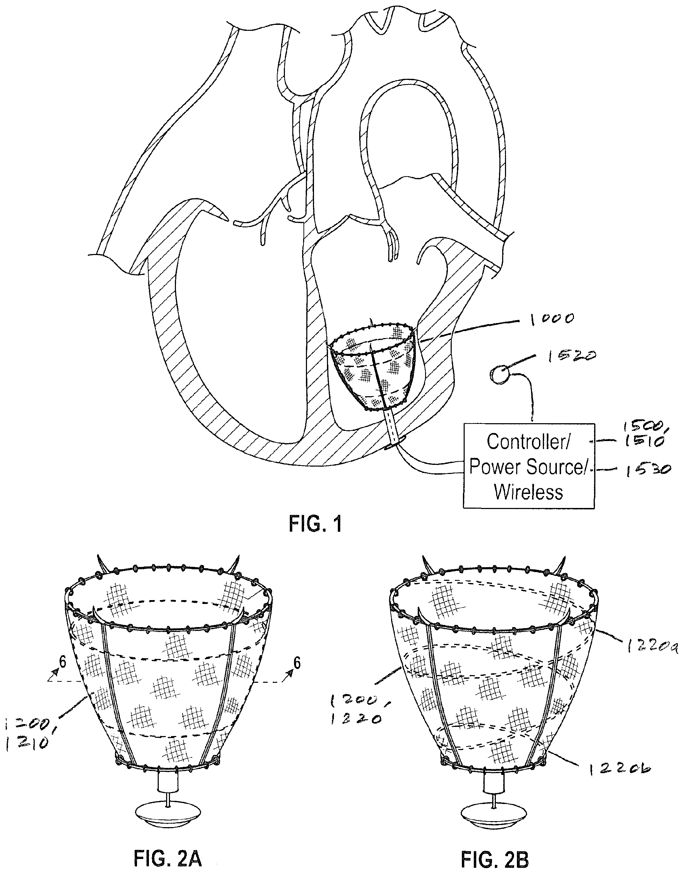

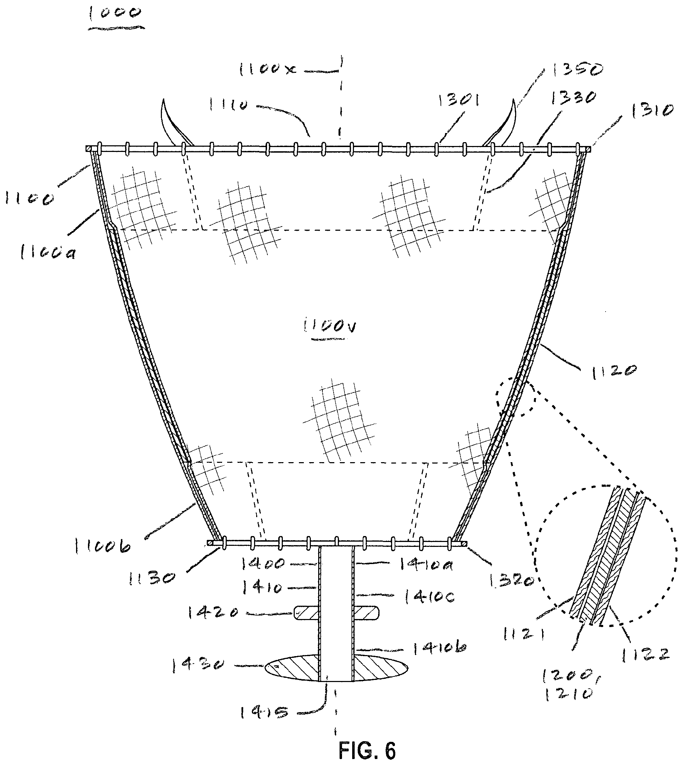

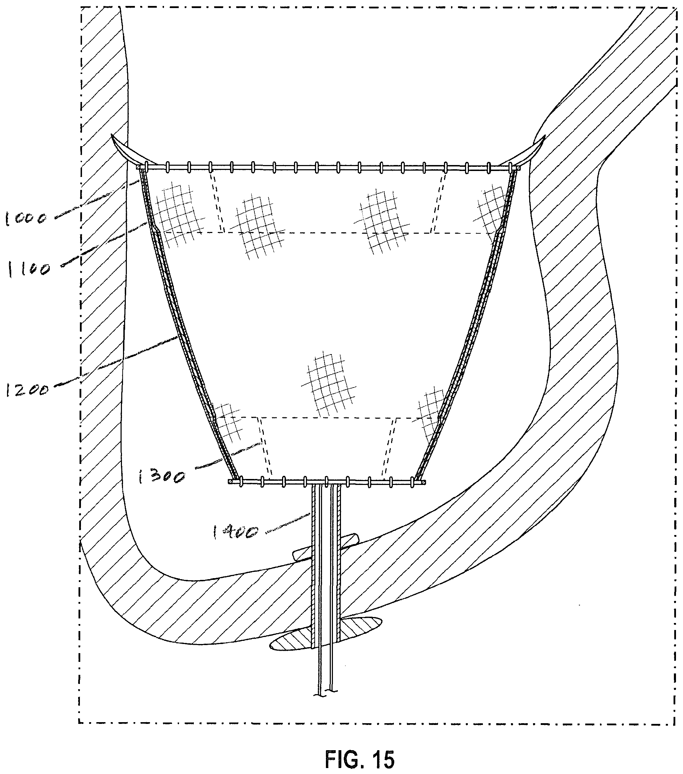

FIGURE 1 shows a ventricular assist device 1000 in place in a left ventricle of the heart. FIGURE 6 shows a vertical cross-sectional view of ventricular assist device 1000. FIGURE 15 shows a vertical cross-sectional view of ventricular assist device 1000 in place in a left ventricle of the heart.

Ventricular assist device 1000 is configured to be placed in a ventricle of the heart. Ventricular assist device 1000 may be configured to be placed in the left ventricle and/or the right ventricle of the heart.

Ventricular assist device 1000 supplements heart function by providing pulsatile flow. Ventricular assist device 1000 is free of rotating elements that may damage red blood cells.

Ventricular assist device 1000 includes a pumping pouch 1100. Pumping pouch 1100 may have a longitudinal axis 1100x.

Pumping pouch 1100 may be flexible. Pumping pouch 1100 may be at least in part impermeable and/or semi-permeable to blood. Pumping pouch 1100 may be at least in part made of an inelastic material and/or an elastic material. Pumping pouch 1100 may be made of expanded polytetrafluoroethylene (ePTFE) and/or any other suitable material.

Pumping pouch 1100 has an opening 1110. Opening 1110 may be circular, elliptical, or any other suitable shape. Opening 1110 may be as wide as the widest part of pumping pouch 1100.

Opening 1110 may be pointed at the aortic valve when ventricular assist device 1000 is placed in the left ventricle. Opening 1110 may be pointed at the pulmonary valve when ventricular assist device 1000 is placed in the right ventricle.

Pumping pouch 1100 defines an internal volume 1100v, which is configured to fill with blood in through opening 1110.

Pumping pouch 1100 may include an upper portion 1100a and a lower portion 1100b. Pumping pouch 1100 may be wider at upper portion 1100a and narrower at lower portion 1100b. Pumping pouch 1100 may be the same width from upper portion 1100a to lower portion 1100b.

Pumping pouch 1100 may have a horizontal cross section that is uniform, or changes in size and/or shape from upper portion 1100a to lower portion 1100b. Pumping pouch 1100 may have a horizontal cross section that is one or more of circular, elliptical, and any other suitable shape.

Pumping pouch 1100 may include a side wall 1120. Side wall 1120 may include an inner layer 1121 and an outer layer 1122.

Pumping pouch 1100 may include a bottom wall 1130. Bottom wall 1130 may include an inner layer 1131 and an outer layer 1132.

Bottom wall 1130 may be flat, rounded, or cone-shaped. Bottom wall 1130 may be parallel to or angled to opening 1110. Bottom wall 1130 may be circular, elliptical, or any other suitable shape.

In one embodiment, pumping pouch 1100 may be shaped like a frustum or a truncated cone, with side wall 1120 that is wider at upper portion 1100a and narrower at lower portion 1100b, and bottom wall 1130 that is flat. Pumping pouch 1100 with lower portion 1100b that is truncated may allow more blood flow under pumping pouch 1100.

In other embodiments, pumping pouch 1100 may be shaped like a funnel, cone, hollow hemisphere, or hollow ellipsoid.

Pumping pouch 1100 may be sized so that even when fully filled it does not touch the walls of the heart. Pumping pouch 1100 may be sized to allow more blood flow between the outside of pumping pouch 1100 and the walls of the heart. This may allow the walls of the heart to be better oxygenated. This may also reduce the likelihood of thrombosis between pumping pouch 1100 and the walls of the heart.

Ventricular assist device 1000 further includes at least one contraction element 1200 coupled to pumping pouch 1100.

Contraction element 1200 is capable of squeezing at least a portion of pumping pouch 1100 to force at least a portion of the blood in internal volume 1100v out through opening 1110. Contraction element 1200 is capable of exerting force inward on at least a portion of pumping pouch 1100 to force at least a portion of the blood in internal volume 1100v out through opening 1110. Contraction element 1200 may be capable of pushing in on side wall 1120 and/or bottom wall 1130 of pumping pouch 1100. Contraction element 1200 may be capable of moving inward.

Contraction element 1200 has an actuated state and an unactuated state. Contraction element 1200 may have an actuated state that is exerting force inward on pumping pouch 1100, and an unactuated state that is not exerting force inward on pumping pouch 1100, or contraction element 1200 may have an unactuated state that is exerting force inward on pumping pouch 1100, and an actuated state is not exerting force inward on pumping pouch 1100.

Contraction element 1200 may include one or more of an electroactive polymer, shape memory alloy, shape memory ceramic, twisted coil alloy, and any other suitable material. Contraction element 1200 may be actuated by electricity.

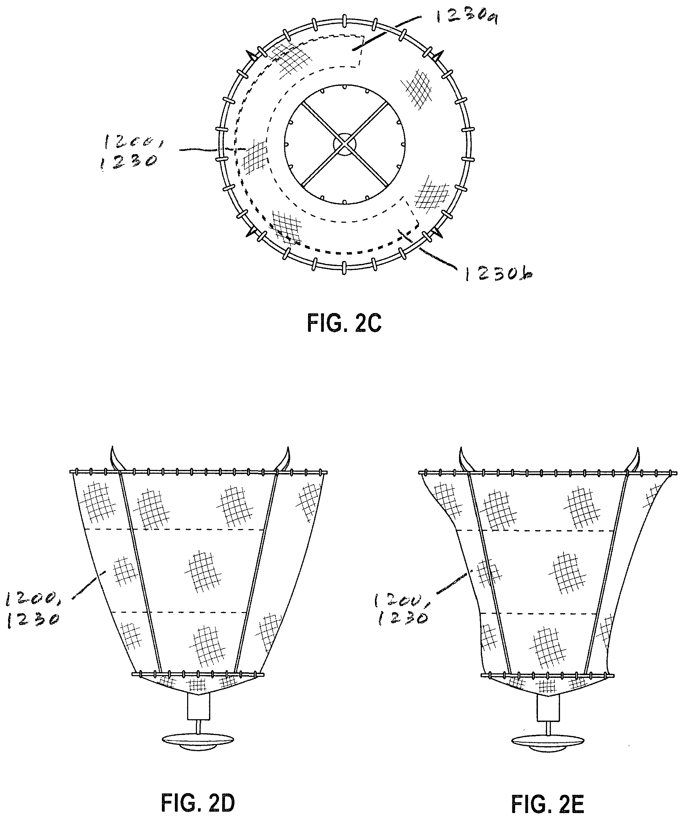

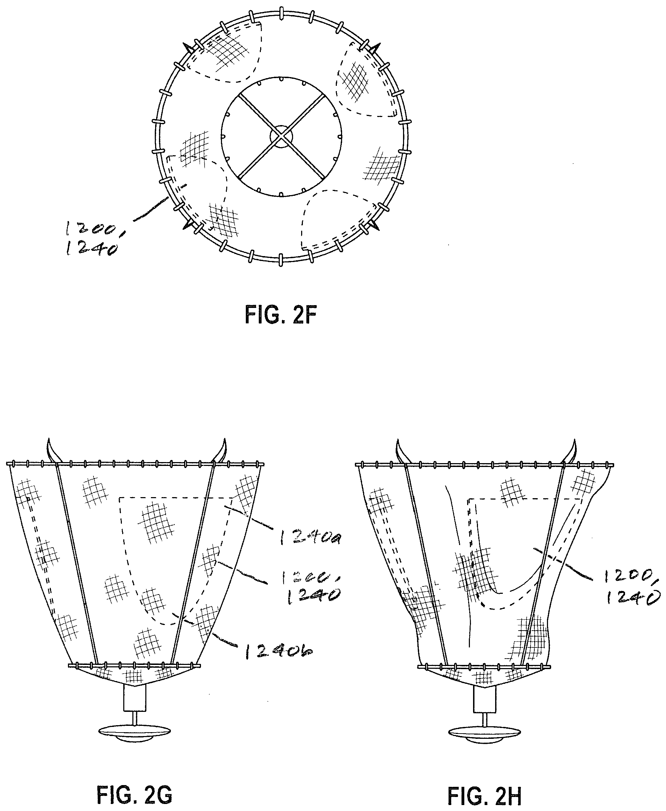

FIGURE 2A shows one embodiment of ventricular assist device 1000 including a band 1210.

Contraction element 1200 may include at least one band 1210. Band 1210 may be coupled to side wall 1120 of pumping pouch 1100. Band 1210 may be coupled to inner layer 1121 and/or outer layer 1122 of side wall 1120. Band 1210 may be coupled between inner layer 1121 and outer layer 1122 of side wall 1120.

Band 1210 may encircle internal volume 1100v.

Band 1210 may be configured to squeeze from a lower portion to an upper portion of band 1210 to push blood up toward opening 1110. Each band 1210 may include one or more separate elements.

Band 1210 may have a uniform or non-uniform width.

FIGURE 2B shows one embodiment of ventricular assist device 1000 including a helix 1220.

Contraction element 1200 may include at least one helix 1220. Helix 1220 may be coupled to side wall 1120 of pumping pouch 1100. Helix 1220 may be coupled to inner layer 1121 and/or outer layer 1122 of side wall 1120. Helix 1220 may be coupled between inner layer 1121 and outer layer 1122 of side wall 1120.

Helix 1220 may wind around internal volume 1100v.

Helix 1220 may impart a twist to pumping pouch 1100 when squeezing blood out of internal volume 1100v. Helix 1220 may supplement or reinforce a vortex in the blood when squeezing blood out of internal volume 1100v. Helix 1220 may wind in a clockwise or counterclockwise direction when viewed from nearer to farther.

Helix 1220 may be configured to squeeze from a lower portion 1220b to an upper portion 1220a of helix 1220 to push blood up toward opening 1110. Each helix 1220 may include one or more separate elements.

Helix 1220 may have a uniform or non-uniform width.

FIGURES 2C-2D show top and side views of one embodiment of ventricular assist device 1000 including a partial band 1230. FIGURE 2E shows partial band 1230 squeezing pumping pouch 1100.

Contraction element 1200 may include at least one partial band 1230.

Partial band 1230 may be coupled to side wall 1120 of pumping pouch 1100. Partial band 1230 may be coupled to inner layer 1121 and/or outer layer 1122 of side wall 1120. Partial band 1230 may be coupled between inner layer 1121 and outer layer 1122 of side wall 1120.

Partial band 1230 may at least partially encircle internal volume 1100v of pumping pouch 1100.

Partial band 1230 may include a first end portion 1230a and a second end portion 1230b. First end portion 1230a may be coupled to pumping pouch 1100. Second end portion 1230b may be free.

Partial band 1230 may be oriented circumferentially and/or at an angle. Second end portion 1230b may extend below first end portion 1230a.

Partial band 1230 may impart a twist to pumping pouch 1100 when squeezing blood out of internal volume 1100v. Partial band 1230 may supplement or reinforce a vortex in the blood when squeezing the blood out of internal volume 1100v. Partial band 1230 may extend in a clockwise or counterclockwise direction from first end portion 1230a to second end portion 1230b when viewed from the top through opening 1110.

Partial band 1230 may be configured to squeeze from second end portion 1230b to first end portion 1230a. Partial band 1230 may be configured to squeeze from a lower portion to an upper portion of partial band 1230 to push blood up toward opening 1110. Each partial band 1230 may include one or more separate elements.

Partial band 1230 may have a uniform or non-uniform width. Second end portion 1230b may be square, round, or any other suitable shape.

FIGURES 2F-2G show top view and side views of one embodiment of ventricular assist device 1000 including a flap 1240. FIGURE 2G shows flap 1240 squeezing pumping pouch 1100.

Contraction element 1200 may include at least one flap 1240. Flap 1240 may be coupled to side wall 1120 of pumping pouch 1100. Flap 1240 may be coupled to inner layer 1121 and/or outer layer 1122 of side wall 1120. Flap 1240 may be coupled between inner layer 1121 and outer layer 1122 of side wall 1120.

Flap 1240 may include a first end portion 1240a and a second end portion 1240b. First end portion 1240a may be coupled to pumping pouch 1100. Second end portion 1240b may be free.

Flap 1240 may be oriented longitudinally, at an angle, or circumferentially. Second end portion 1240b may extend below first end portion 1240a.

Flap 1240 may impart a twist to pumping pouch 1100 when squeezing blood out of internal volume 1100v. Flap 1240 may supplement or reinforce a vortex in the blood when squeezing the blood out of internal volume 1100v. Flap 1240 may extend in a clockwise or counterclockwise direction from first end portion 1240a to second end portion 1240b when viewed from the top through opening 1110.

Flap 1240 may be configured to squeeze from second end portion 1240b to first end portion 1240a. Flap 1240 may be configured to squeeze from a lower portion to an upper portion of flap 1240 to push blood up toward opening 1110. Each flap 1240 may include one or more separate elements.

Flap 1240 may have a non-uniform or uniform width. Second end portion 1240b may be round, square, or any other suitable shape.

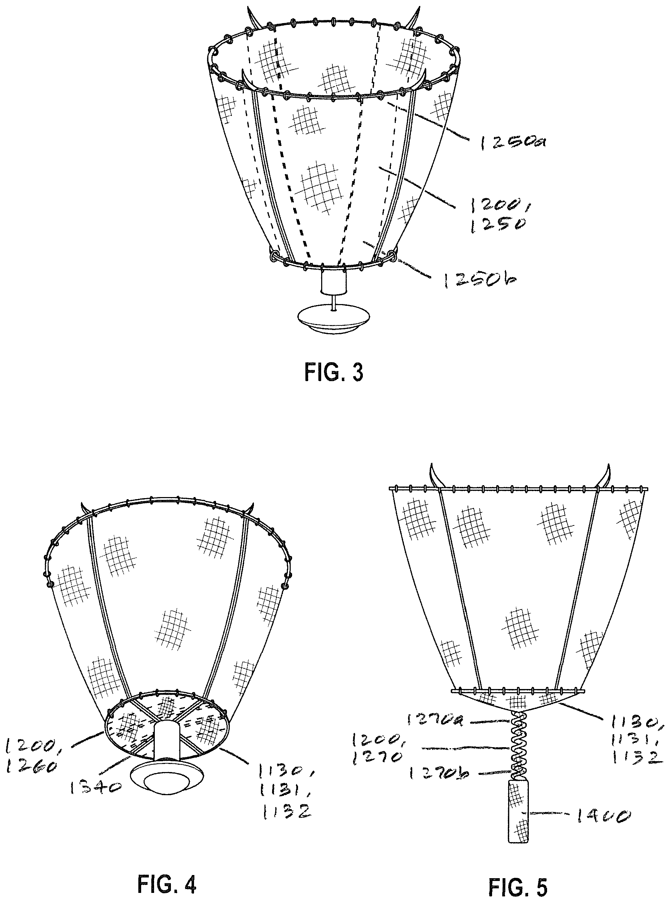

FIGURE 3 shows one embodiment of ventricular assist device 1000 including a side strip 1250.

Contraction element 1200 may include at least one side strip 1250. Side strip 1250 may be coupled to side wall 1120 of pumping pouch 1100. Side strip 1250 may be coupled to inner layer 1121 and/or outer layer 1122 of side wall 1120. Side strip 1250 may be coupled between inner layer 1121 and outer layer 1122 of side wall 1120. Side strip 1250 may be coupled to side wall 1120 along at least a part of a length of side strip 1250. Side strip 1250 may extend from upper portion 1100a to lower portion 1100b of pumping pouch 1100.

Side strip 1250 may be oriented longitudinally and/or at an angle. Side strip 1250 with an angled orientation may impart a twist to pumping pouch 1100 when squeezing the blood out of internal volume 1100v. Side strip 1250 may supplement or reinforce a vortex in the blood when squeezing blood out of internal volume 1100v. Side strip 1250 may be angled in a clockwise or counterclockwise direction when viewed from nearer to farther.

Side strip 1250 may be configured to squeeze from a lower portion 1250b to an upper portion 1250a of side strip 1250 to push blood up toward opening 1110. Each side strip 1250 may include one or more separate elements.

Side strip 1250 may have a uniform or non-uniform width. Side strip 1250 may be one or more of straight, curved, wavy, meandering, and any other suitable shape.

FIGURE 4 shows one embodiment of ventricular assist device 1000 including a bottom strip 1260.

Contraction element 1200 may include at least one bottom strip 1260. Bottom strip 1260 may be coupled to bottom wall 1130 of pumping pouch 1100. Bottom strip 1260 may be coupled to inner layer 1131 and/or outer layer 1132 of bottom wall 1130. Bottom strip 1260 may be coupled between inner layer 1131 and outer layer 1132 of bottom wall 1130.

Bottom strip 1260 may extend across at least a portion of bottom wall 1130 of pumping pouch 1100. Bottom strip 1260 may be arranged parallel to and/or crossing any other bottom strips 1260.

Bottom strip 1260 may be configured to push up bottom wall 1130 to force blood out of pumping pouch 1100.

Bottom strip 1260 may have a uniform or non-uniform width. Bottom strip 1260 may be one or more of straight, curved, wavy, meandering, circular, elliptical, and any other suitable shape.

FIGURE 5 shows one embodiment of ventricular assist device 1000 including a spring 1270.

Contraction element 1200 may include at least one spring 1270. Spring 1270 may be coupled to bottom wall 1130 and/or side wall 1120 of pumping pouch 1100.

Spring 1270 may include a first end portion 1270a and a second end portion 1270b. First end portion 1270a may be coupled to bottom wall 1130 and/or side wall 1120 of pumping pouch 1100. Second end portion 1270b may be coupled to one or more of a wall of the heart, frame 1300, and stem 1400.

Spring 1270 may be configured to push up bottom wall 1130 and/or push in side wall 1120 to force blood out of pumping pouch 1100.

Ventricular assist device 1000 further includes a frame 1300 coupled to pumping pouch 1100. Frame 1300 may be coupled to pumping pouch 1100 with sutures 1301. Frame 1300 may be coupled to pumping pouch 1100 with one or more of clips, adhesives, and any other suitable devices.

Frame 1300 may include a tubular or ring-like structure. Frame 1300 may be configured to be coupled between the walls of the heart and at least a portion of pumping pouch 1100.

Frame 1300 may include an open structure. Frame 1300 may allow blood to flow between an interior and an exterior of frame 1300.

Frame 1300 is configured to be coupled to the walls of the heart. Frame 1300 may reduce the number of tissue anchors 1350 needed to attach pumping pouch 1100 to the walls of the heart.

Frame 1300 may provide support to pumping pouch 1100. Frame 1300 may help keep opening 1110 open, and may help prevent opening 1110 from collapsing. Frame 1300 may help keep pumping pouch 1100 unfurled, and may help prevent pumping pouch 1100 from inverting.

Frame 1300 may help orient pumping pouch 1100. For example, frame 1300 may point opening 1110 towards the aortic valve when ventricular assist device 1000 is placed in the left ventricle. As another example, frame 1300 may point opening 1110 towards the pulmonary valve when ventricular assist device 1000 is placed in the right ventricle.

Frame 1300 may be sized so that it does not touch the walls of the heart when in place. Frame 1300 may be sized so that there is a space of approximately 1 mm to 2 mm between frame 1300 and the walls of the heart.

Frame 1300 may help to maintain separation between pumping pouch 1100 and the walls of the heart. Frame 1300 may allow more blood flow between the outside of pumping pouch 1100 and the walls of the heart. This may allow the walls of the heart to be better oxygenated. This may also reduce the likelihood of thrombosis between pumping pouch 1100 and the walls of the heart.

Frame 1300 may be flexible. Frame 1300 may be made of one or more of a shape memory metal, plastic, and any other suitable material.

Frame 1300 may include an upper ring 1310. Upper ring 1310 may be coupled to upper portion 1100a of pumping pouch 1100. Upper ring 1310 may be coupled to pumping pouch 1100 along an edge of opening 1110. Upper ring 1310 may be circular, elliptical, or any other suitable shape. Upper ring 1310 may be the same or different size and/or shape as opening 1110.

Upper ring 1310 may provide support to opening 1110. Upper ring 1310 may help keep opening 1110 open, and may help prevent opening 1110 from collapsing.

Frame 1300 may include a lower ring 1320. Lower ring 1320 may be coupled to lower portion 1100b of pumping pouch 1100. Lower ring 1320 may be coupled to pumping pouch 1100 along an edge of bottom wall 1130. Lower ring 1320 may be circular, elliptical, or any other suitable shape. Lower ring 1320 may be the same or different size and/or shape as bottom wall 1130 of pumping pouch 1100.

Lower ring 1320 may help keep pumping pouch 1100 unfurled, and may help prevent pumping pouch 1100 from inverting.

Frame 1300 may include at least one side strut 1330. Side strut 1330 may be coupled to upper ring 1310 and/or lower ring 1320. Side strut 1330 may couple upper ring 1310 and lower ring 1320. Side strut 1330 may be straight, curved, or any other suitable shape.

Frame 1300 may include at least one bottom strut 1340. Bottom strut 1340 may be coupled to lower ring 1320. Bottom strut 1340 may be arranged parallel to and/or crossing any other bottom struts 1340.

Frame 1300 may include one or more tissue anchors 1350. Tissue anchors 1350 may be coupled to one or more of upper ring 1310, lower ring 1320, and side strut 1330. Tissue anchors 1350 may be oriented outwards. Tissue anchors 1350 may include one or more of hooks, barbs, adhesives, and any other suitable devices. Tissue anchors 1350 may be configured to attach one or more of upper ring 1310, lower ring 1320, and struts 1330 to the walls of the heart.

Tissue anchors 1350 may be configured to maintain separation between frame 1300 and the walls of the heart.

Frame 1300 may be collapsible for delivery inside catheter 1610 of delivery device 1600. Frame 1300 may be configured to expand when pushed out from catheter 1610 of delivery device 1600. Frame 1300 may help pumping pouch 1100 unfurl during delivery.

Frame 1300 may allow a first pumping pouch to be removed and replaced with a second pumping pouch. Frame 1300 may allow a first pumping pouch to remain in place and a second pumping pouch placed over, under, or around the first pumping pouch.

Ventricular assist device 1000 may include a stem 1400. Stem 1400 may be coupled to frame 1300.

Stem 1400 may be configured to be coupled to a wall of the heart. Stem 1400 may be configured to be coupled at or near the apex of the heart. Stem 1400 may help anchor and/or position ventricular assist device 1000 in the heart.

Stem 1400 may be flexible. Stem 1400 may be made of one or more of metal, plastic, and any other suitable material.

Stem 1400 may include a body 1410. Body 1410 may include an upper portion 1410a, a central portion 1410c, and a lower portion 1410b. Upper portion 1410a may be coupled to frame 1300. Upper portion 1410a may be coupled to lower ring 1320 and/or bottom strut 1340.

Body 1410 may include a stem lumen 1415. Stem lumen 1415 may be configured to allow control wires and/or power wires to pass through.

Body 1410 may be configured to be attached to a wall of the heart. Body 1410 may be configured to pass at least partially through a wall of the heart. Body 1410 may be long enough to pass through a wall of the heart.

Stem 1400 may include an inner stop 1420. Inner stop 1420 may be coupled to a central portion 1410c of body 1410. Inner stop 1420 may help keep stem 1400 in place. Inner stop 1420 may help reduce or prevent blood leaking out around body 1410.

Stem 1400 may include a outer stop 1430. Outer stop 1430 may be coupled to a lower portion 1410b of body 1410. Outer stop 1430 may help keep stem 1400 properly positioned.

Ventricular assist device 1000 also includes a controller 1500 electrically coupled to contraction element 1200, which may be wired and/or wirelessly.

Controller 1500 is configured to control actuation of contraction element 1200 and is configured to synchronize actuation and/or deactuation of contraction element 1200 with the beating of the heart. Controller 1500 may be configured to synchronization actuation and/or deactuation of contraction element 1200 with the contraction of the ventricles.

Controller 1500 includes a processor 1510, which is configured to use data from a sensor 1520 and provide an actuation signal to contraction element 1200.

Controller 1500 may include at least one sensor 1520. Sensor 1520 may be electrically coupled to processor 1510. Sensor 1520 is configured to sense the beating of the heart. Sensor 1520 may include one or more of electrical, pressure, and any other suitable sensor.

Controller 1500 may include a power source 1530. Power source 1530 may be electrically coupled to processor 1510.

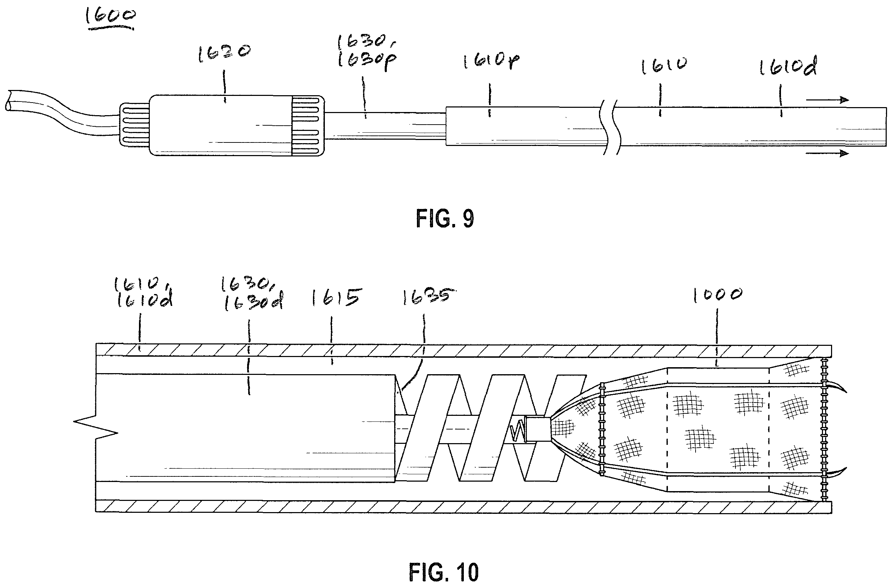

FIGURE 9 shows one embodiment of a delivery device 1600. FIGURE 10 shows a cross-sectional view of delivery device 1600, with ventricular assist device 1000 collapsed into a delivery configuration.

Delivery device 1600 may be used to place ventricular assist device 1000 in the heart. Delivery device 1600 may be configured to accept ventricular assist device 1000 collapsed into a delivery configuration.

Delivery device 1600 may include a catheter 1610. Catheter 1610 may include a proximal portion 1610p and a distal portion 1610d.

Catheter 1610 may include a catheter lumen 1615. Catheter lumen 1615 may be configured to accept ventricular assist device 1000 collapsed into a delivery configuration. Ventricular assist device 1000 may be placed in catheter lumen 1615 at or near distal portion 1610d of catheter 1610.

Delivery device 1600 may include a handle 1620. Handle 1620 may be coupled to proximal portion 1610p of catheter 1610.

Delivery device 1600 may include a pusher 1630. Pusher 1630 may be coupled to catheter 1610 and/or handle 1620. Pusher 1630 may be slidingly and/or threadingly coupled inside catheter lumen 1615. Pusher 1630 may include a proximal portion 1630p and a distal portion 1630d. Pusher 1630 may include a pusher lumen 1635. Pusher lumen 1635 may be configured to allow control wires and/or power wires from stem 1400 to pass through.

Pusher 1630 may be configured to push ventricular assist device 1000 out of catheter 1610. Distal portion 1630d of pusher may push against one or more of pumping pouch 1100, frame 1300, and stem 1400 to push ventricular assist device 1000 out of catheter lumen 1615 at distal end 1610d of catheter 1610. Pusher 1630 may be advanced through catheter lumen 1615 by pushing and/or turning proximal portion 163 Op of pusher 1630.

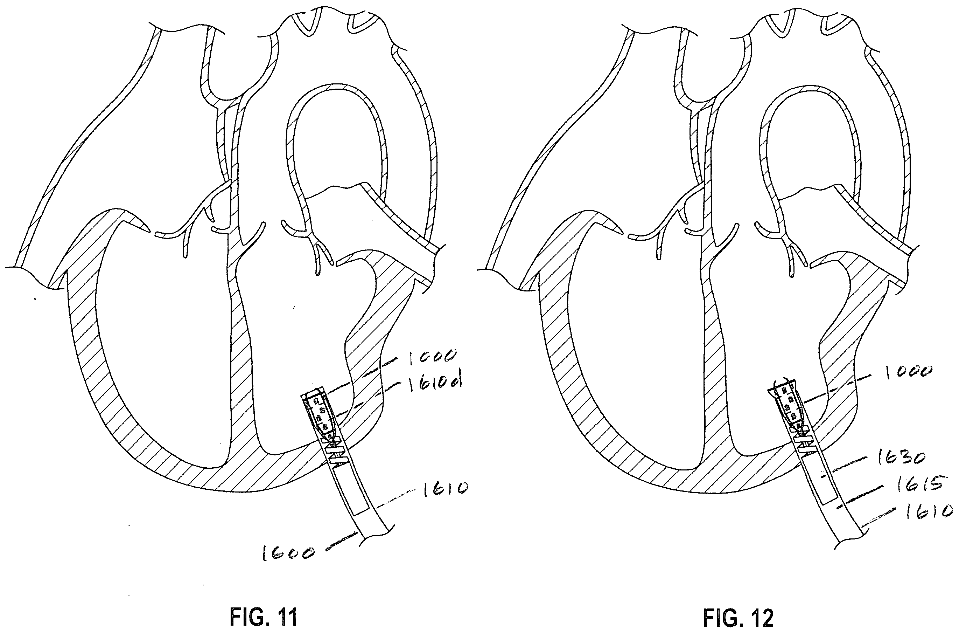

FIGURES 11-14 show one embodiment of a method for delivering ventricular assist device 1000 using delivery device 1600.

FIGURE 11 shows introducing catheter 1610 of delivery device 1600 into the left ventricle transapically. Catheter 1610 is advanced through the apex, and distal portion 1610d of catheter 1610 is positioned in the left ventricle.

FIGURE 12 shows pushing ventricular assist device 1000 out of catheter 1610. Pusher 1630 is advanced through catheter lumen 1615 to push ventricular assist device 1000 out of catheter lumen 1615.

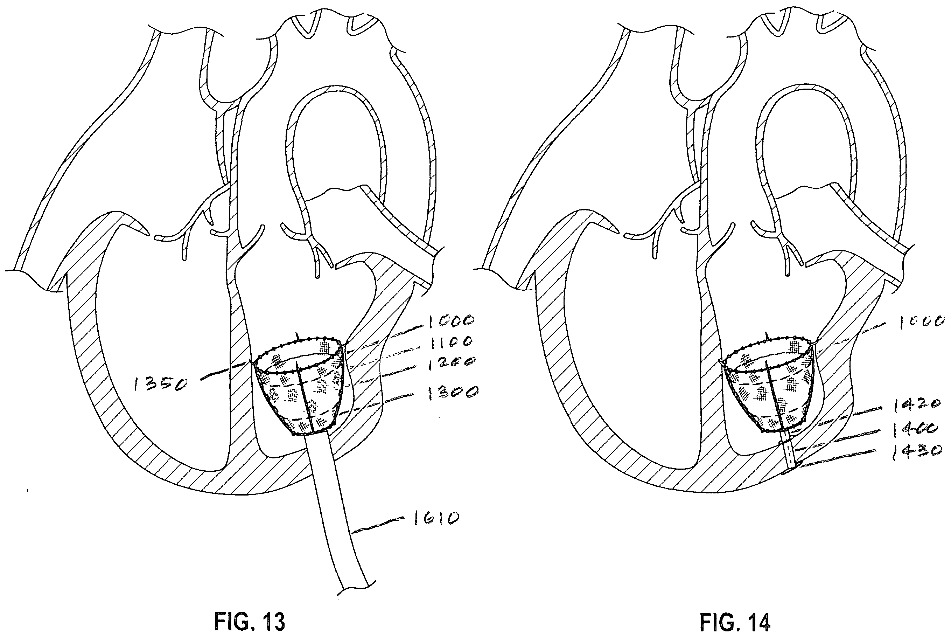

FIGURE 13 shows allowing ventricular assist device 1000 to expand inside the left ventricle. Pumping pouch 1100, contraction element 1200, and frame 1300 expand as they are pushed out of catheter 1610. Tissue anchors 1350 attach to the walls of the heart.

FIGURE 14 shows withdrawing catheter 1610 to leave ventricular assist device 1000 in place. Catheter 1610 is withdrawn, leaving stem 1400 positioned through the wall of the heart, with inner stop 1420 inside the left ventricle and outer stop 1430 outside the heart, and control wires and/or power wires leading out of stem 1400.

Ventricular assist device 1000 may also be delivered percutaneously, for example, through the aortic valve, or with an open procedure.

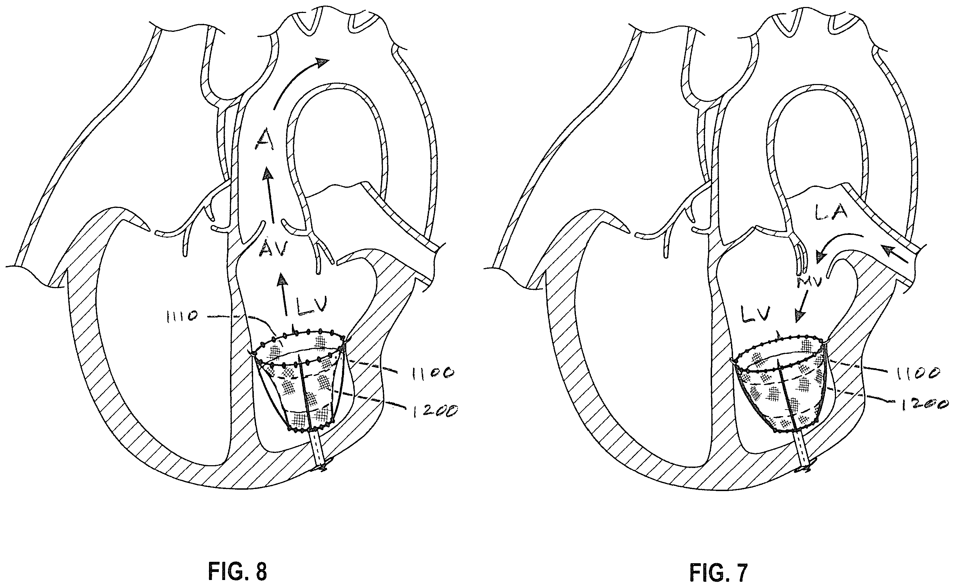

FIGURES 7-8 show ventricular assist device 1000 in place in a left ventricle of the heart, with the heart in diastole and systole, respectively.

FIGURE 7 shows the heart in diastole. The left atrium LA contracts, pumping blood from the left atrium LA through the mitral valve MV and into the left ventricle LV. In addition, the left ventricle LV is relaxed, which creates a suction that draws blood from the left atrium LA into the left ventricle LV. Pumping pouch 1100 is not being squeezed by contraction element 1200, which may also create a suction that helps draw blood from the left atrium LA into the left ventricle LV, and may improve filling of the left ventricle LV.

FIGURE 8 shows the heart in systole. The left ventricle LV contracts, pumping blood from the left ventricle LV through the aortic valve AV and into the aorta A. Pumping pouch 1100 is squeezed by contraction element 1200, which also pumps blood from the left ventricle LV into the aorta A. Pumping pouch 1100 may be squeezed in synchronization with the contraction of the left ventricle LV. Opening 1110 may be pointed at the aortic valve AV.

## Claims

1. A ventricular assist device (1000) configured to be placed in a ventricle of a heart, the ventricular assist device (1000) comprising:
a pumping pouch (1100) having an opening (1110) and defining an internal volume (1100v) configured to fill with blood in through the opening (1110);
a contraction element (1200) coupled to the pumping pouch (1100), the contraction element (1200) being actuatable upon receipt of an actuation signal between first and second states in which force is exerted or not exerted inward on the pumping pouch (1100) such that when force is exerted inward on the pumping pouch (1100), at least a portion of the pumping pouch (1100) is squeezed to force at least a portion of the blood out through the opening (1110);
a frame (1300) including an upper ring (1310) coupled to the pumping pouch (1100) along an edge of the opening (1110), the frame (1300) configured to be coupled to a wall of the heart; and
a controller (1500) electrically coupled to the contraction element (1200), the controller (1500) comprising a processor (1510) configured to receive, in use, a signal from a sensor (1520) configured to sense beating of the heart and to provide the actuation signal to the contraction element (1200) to synchronize actuation of the contraction element (1200) between the first and second states with the beating of the heart.

2. The ventricular assist device of claim 1, wherein the contraction element includes an electroactive polymer

3. The ventricular assist device of claim 1, wherein the contraction element includes one or more of:
a shape memory alloy;
shape memory ceramic; and
a twisted coil alloy.

4. The ventricular assist device of claim 1, wherein the contraction element is actuated by electricity.

5. The ventricular assist device of claim 1, wherein the contraction element includes a circumferential band coupled to a side wall of the pumping pouch, the circumferential band encircling the internal volume.

6. The ventricular assist device of claim 1, wherein the contraction element includes a helix coupled to a side wall of the pumping pouch, the helix winding around the internal volume.

7. The ventricular assist device of claim 1, wherein the contraction element includes a partial band coupled to a side wall of the pumping pouch, the partial band at least partially encircling the internal volume.

8. The ventricular assist device of claim 1, wherein the contraction element includes a flap coupled to a side wall of the pumping pouch, the flap having a first end portion coupled to the pumping pouch, the flap having a second end portion that is free.

9. The ventricular assist device of claim 1, wherein the contraction element includes a side strip coupled to a side wall of the pumping pouch, the side strip extending from an upper portion of the pumping pouch to a lower portion of the pumping pouch or wherein the contraction element includes a bottom strip coupled to a bottom wall of the pumping pouch.

10. The ventricular assist device of claim 1, wherein the contraction element includes a spring coupled to a bottom wall of the pumping pouch.

11. The ventricular assist device of claim 1, wherein the frame includes an upper ring coupled to an upper portion of the pumping pouch, a lower ring coupled to a lower portion of the pumping pouch, and one or more struts coupling the upper ring to the lower ring.

12. The ventricular assist device of claim 1, wherein the frame includes one or more tissue anchors configured to be coupled to the wall of the heart.

13. The ventricular assist device of claim 1, wherein the pumping pouch is impermeable to blood.

14. The ventricular assist device of claim 1, wherein the pumping pouch is made of an inelastic material.

15. The ventricular assist device of claim 1, further comprising:
a stem coupled to the pumping pouch, the stem configured to be coupled to the wall of the heart.

## Patentansprüche

1. Ventrikuläres Unterstützungssystem (1000), ausgebildet, in einem Ventrikel eines Herzens platziert zu werden, wobei das ventrikuläre Unterstützungssystem (1000) umfasst:
ein Pumpbeutel (1100) mit einer Öffnung (1110), der ein Innenvolumen (1100v) definiert, das so ausgebildet ist, dass es sich durch die Öffnung (1110) mit Blut füllt;
ein Kontraktionselement (1200), gekoppelt an den Pumpbeutel (1100), wobei das Kontraktionselement (1200) bei Empfang eines Betätigungssignals zwischen erstem und zweitem Zustand, wobei eine Kraft nach innen auf den Pumpbeutel (1100) ausgeübt wird oder nicht ausgeübt wird, betätigt werden kann, derart, dass, wenn eine Kraft nach innen auf den Pumpbeutel (1100) ausgeübt wird, zumindest ein Abschnitt des Pumpbeutels (1100) zusammengedrückt wird, um zumindest einen Abschnitt des Blutes durch die Öffnung (1110) nach außen zu drücken;
einen Rahmen (1300), einschließlich eines oberen Rings (1310), der entlang einer Kante der Öffnung (1110) an den Pumpbeutel (1100) gekoppelt ist, wobei der Rahmen (1300) so ausgebildet ist, dass er mit einer Herzwand koppelbar ist; und
eine Steuereinheit (1500), elektrisch an das Kontraktionselement (1200) gekoppelt, wobei die Steuereinheit (1500) einen Prozessor (1510) umfasst, der so ausgebildet ist, bei Gebrauch ein Signal von einem Sensor (1520), der so ausgebildet ist, dass er ein Schlagen des Herzens erfasst, zu empfangen und das Betätigungssignal an das Kontraktionselement (1200) bereitzustellen, um eine Betätigung des Kontraktionselements (1200) zwischen ersten und zweiten Zustand mit dem Schlagen des Herzens zu synchronisieren.

2. Ventrikuläres Unterstützungssystem nach Anspruch 1, wobei das Kontraktionselement ein elektroaktives Polymer einschließt

3. Ventrikuläres Unterstützungssystem nach Anspruch 1, wobei das Kontraktionselement eines oder mehrere der folgenden einschließt:
eine Formgedächtnislegierung;
Formgedächtniskeramik; und
eine verdrillte Spulenlegierung.

4. Ventrikuläres Unterstützungssystem nach Anspruch 1, wobei das Kontraktionselement durch Elektrizität betätigt wird.

5. Ventrikuläres Unterstützungssystem nach Anspruch 1, wobei das Kontraktionselement ein umfangsverlaufendes Band einschließt, das mit einer Seitenwand des Pumpbeutels gekoppelt ist, wobei das umfangsverlaufende Band das Innenvolumen umschließt.

6. Ventrikuläres Unterstützungssystem nach Anspruch 1, wobei das Kontraktionselement eine Helix einschließt, die mit einer Seitenwand des Pumpbeutels gekoppelt ist, wobei die Helix um das Innenvolumen herum gewunden ist.

7. Ventrikuläres Unterstützungssystem nach Anspruch 1, wobei das Kontraktionselement ein Teilband einschließt, das mit einer Seitenwand des Pumpbeutels gekoppelt ist, wobei das Teilband zumindest teilweise das Innenvolumen umschließt.

8. Ventrikuläres Unterstützungssystem nach Anspruch 1, wobei das Kontraktionselement eine Lasche einschließt, die mit einer Seitenwand des Pumpbeutels gekoppelt ist, wobei die Lasche einen ersten Endabschnitt aufweist, der an den Pumpbeutel gekoppelt ist, wobei die Lasche einen zweiten Endabschnitt aufweist, der frei ist.

9. Ventrikuläres Unterstützungssystem nach Anspruch 1, wobei das Kontraktionselement einen Seitenstreifen einschließt, der mit einer Seitenwand des Pumpbeutels gekoppelt ist, wobei sich der Seitenstreifen von einem oberen Abschnitt des Pumpbeutels zu einem unteren Abschnitt des Pumpbeutels erstreckt, oder wobei das Kontraktionselement einen Bodenstreifen einschließt, der mit einer Bodenwand des Pumpbeutels gekoppelt ist.

10. Ventrikuläres Unterstützungssystem nach Anspruch 1, wobei das Kontraktionselement eine Feder einschließt, die mit einer Bodenwand des Pumpbeutels gekoppelt ist.

11. Ventrikuläres Unterstützungssystem nach Anspruch 1, wobei der Rahmen einen oberen Ring, der mit einem oberen Abschnitt des Pumpbeutels gekoppelt ist, einen unteren Ring, der mit einem unteren Abschnitt des Pumpbeutels gekoppelt ist, und eine oder mehrere Streben, die den oberen Ring an den unteren Ring koppeln, einschließt.

12. Ventrikuläres Unterstützungssystem nach Anspruch 1, wobei der Rahmen einen oder mehrere Gewebeanker einschließt, die so ausgebildet sind, dass sie mit der Herzwand koppelbar sind.

13. Ventrikuläres Unterstützungssystem nach Anspruch 1, wobei der Pumpbeutel für Blut undurchlässig ist.

14. Ventrikuläres Unterstützungssystem nach Anspruch 1, wobei der Pumpbeutel aus einem unelastischen Material hergestellt ist.

15. Ventrikuläres Unterstützungssystem nach Anspruch 1, weiter umfassend:
einen Stiel, der mit dem Pumpbeutel gekoppelt ist, wobei der Stiel so ausgebildet ist, dass er mit der Herzwand koppelbar ist.

## Revendications

1. Dispositif d'assistance ventriculaire (1000) configuré pour être placé dans un ventricule d'un cœur, le dispositif d'assistance ventriculaire (1000) comprenant :
une poche de pompage (1100) présentant une ouverture (1110) et définissant un volume interne (1100v) configuré pour se remplir de sang à travers l'ouverture (1110) ;
un élément de contraction (1200) couplé à la poche de pompage (1100), l'élément de contraction (1200) étant actionnable à la réception d'un signal d'actionnement entre des premier et deuxième états dans lesquels une force est exercée ou non exercée vers l'intérieur sur la poche de pompage (1100) de sorte que lorsque la force est exercée vers l'intérieur sur la poche de pompage (1100), au moins une partie de la poche de pompage (1100) est comprimée pour forcer au moins une partie du sang à sortir par l'ouverture (1110) ;
un cadre (1300) incluant un anneau supérieur (1310) couplé à la poche de pompage (1100) le long d'un bord de l'ouverture (1110), le cadre (1300) étant configuré pour être couplé à une paroi du cœur ; et
un dispositif de commande (1500) couplé électriquement à l'élément de contraction (1200), le dispositif de commande (1500) comprenant un processeur (1510) configuré pour recevoir, lors d'une utilisation, un signal d'un capteur (1520) configuré pour détecter le battement du cœur et pour fournir le signal d'actionnement à l'élément de contraction (1200) pour synchroniser l'actionnement de l'élément de contraction (1200) entre les premier et deuxième états avec le battement du cœur.

2. Dispositif d'assistance ventriculaire selon la revendication 1, dans lequel l'élément de contraction inclut un polymère électroactif.

3. Dispositif d'assistance ventriculaire selon la revendication 1, dans lequel l'élément de contraction inclut un ou plusieurs parmi :
un alliage à mémoire de forme ;
une céramique à mémoire de forme ; et
un alliage de bobines torsadées.

4. Dispositif d'assistance ventriculaire selon la revendication 1, dans lequel l'élément de contraction est actionné par l'électricité.

5. Dispositif d'assistance ventriculaire selon la revendication 1, dans lequel l'élément de contraction inclut une bande circonférentielle couplée à une paroi latérale de la poche de pompage, la bande circonférentielle entourant le volume interne.

6. Dispositif d'assistance ventriculaire selon la revendication 1, dans lequel l'élément de contraction inclut une hélice couplée à une paroi latérale de la poche de pompage, l'hélice s'enroulant autour du volume interne.

7. Dispositif d'assistance ventriculaire selon la revendication 1, dans lequel l'élément de contraction inclut une bande partielle couplée à une paroi latérale de la poche de pompage, la bande partielle entourant au moins partiellement le volume interne.

8. Dispositif d'assistance ventriculaire selon la revendication 1, dans lequel l'élément de contraction inclut un volet couplé à une paroi latérale de la poche de pompage, le volet présentant une première partie d'extrémité couplée à la poche de pompage, le volet présentant une deuxième partie d'extrémité qui est libre.

9. Dispositif d'assistance ventriculaire selon la revendication 1, dans lequel l'élément de contraction inclut une bande latérale couplée à une paroi latérale de la poche de pompage, la bande latérale s'étendant d'une partie supérieure de la poche de pompage à une partie inférieure de la poche de pompage ou dans lequel l'élément de contraction inclut une bande inférieure couplée à une paroi inférieure de la poche de pompage.

10. Dispositif d'assistance ventriculaire selon la revendication 1, dans lequel l'élément de contraction inclut un ressort couplé à une paroi inférieure de la poche de pompage.

11. Dispositif d'assistance ventriculaire selon la revendication 1, dans lequel le cadre inclut un anneau supérieur couplé à une partie supérieure de la poche de pompage, un anneau inférieur couplé à une partie inférieure de la poche de pompage, et une ou plusieurs entretoises couplant l'anneau supérieur à l'anneau inférieur.

12. Dispositif d'assistance ventriculaire selon la revendication 1, dans lequel le cadre inclut une ou plusieurs ancres tissulaires configurées pour être couplées à la paroi du cœur.

13. Dispositif d'assistance ventriculaire selon la revendication 1, dans lequel la poche de pompage est imperméable au sang.

14. Dispositif d'assistance ventriculaire selon la revendication 1, dans lequel la poche de pompage est constituée d'un matériau non élastique.

15. Dispositif d'assistance ventriculaire selon la revendication 1, comprenant en outre :
une tige couplée à la poche de pompage, la tige étant configurée pour être couplée à la paroi du cœur.
